(19) Europäisches Patentamt
European Patent Office
Office européen des brevets

(11) Veröffentlichungsnummer: **0 011 670**
**A2**

# (12) EUROPÄISCHE PATENTANMELDUNG

(21) Anmeldenummer: **79101296.6**

(22) Anmeldetag: **30.04.79**

(51) Int. Cl.³: **C 07 C 127/15**
C 07 C 125/063, C 07 C 155/03
C 07 C 97/065, C 07 C 101/38
C 07 C 103/30, C 07 C 95/02
C 07 C 69/74, C 07 C 47/43
C 07 C 119/045

(30) Priorität: **30.11.78 DE 2851755**

(43) Veröffentlichungstag der Anmeldung:
**11.06.80 Patentblatt 80/12**

(84) Benannte Vertragsstaaten:
**BE CH DE FR GB IT NL**

(71) Anmelder: **BAYER AG**
**Zentralbereich Patente, Marken und Lizenzen**
**D-5090 Leverkusen 1, Bayerwerk(DE)**

(72) Erfinder: **Vogel, Emanuel, Prof.Dr.**
**Raschdorffstrasse 7**
**D-5000 Köln 41(DE)**

(72) Erfinder: **Schäfer-Ridder, Maria, Dr.**
**Broichweg 22**
**D-5014 Kerpen(DE)**

(72) Erfinder: **Wagner, Arwed, Dipl-Chem.**
**Bonnstrasse 528**
**D-5030 Hürth(DE)**

(72) Erfinder: **Schwamborn, Michael, Dipl-Chem.**
**Lindenstrasse 16**
**D-5830 Schwelm(DE)**

(72) Erfinder: **Gato, Rodrigues Alexandre, Dipl.-Chem.**
**Auf dem Kölnberg 4/704**
**D-5000 Köln 50(DE)**

**(54) Cycloheptatrien-Derivate und Verfahren zu ihrer Herstellung.**

(57) Die Erfindung betrifft Cycloheptatrien-Derivate der Formel

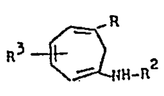,

in der
R für Wasserstoff, $COR^1$ oder $NHR^2$ steht,
$R^1$ Alkyl, Cycloalkyl, Alkoxy oder Cycloalkoxy bedeutet,
$R^2$ für $CONH_2$, $COOR^4$, $CO\text{-}NHR^4$, $CO\text{-}NR^4R^5$, $COSR^4$ oder $COR^4$ steht und für den Fall, daß R $COR^1$ bedeutet, außerdem noch Wasserstoff sein kann,
$R^3$ Wasserstoff, Alkyl oder Cycloalkyl bedeutet und
$R^4$ und $R^5$ gleich oder verschieden sind und Alkyl, Cycloalkyl, Aryl oder Aralkyl bedeuten können.

Die Erfindung betrifft ferner ein Verfahren zur Herstellung dieser neuen Verbindungen, bei dem eine gegebenenfalls wie angegeben substituierten Cycloheptatrien-carbonsäure
a) mit einem Halogenierungsmittel,
b) anschließend mit einem Azid umgesetzt wird,
c) das dabei erhaltene Zwischenprodukt in einem Lösungsmittel unter Feuchtigkeitsausschluß erwärmt wird und
d) das dabei erhaltene Zwischenprodukt mit einer Verbindung mit einem aciden Wasserstoffatom umgesetzt wird.

Die Erfindung betrifft auch die neuen Zwischenprodukte des erfindungsgemäßen Verfahrens.

EP 0 011 670 A2

BAYER AKTIENGESELLSCHAFT 5090 Leverkusen, Bayerwerk
Zentralbereich
Patente, Marken und Lizenzen Ha-kl

Cycloheptatrien-Derivate und Verfahren zu ihrer Herstellung

Die vorliegende Erfindung betrifft neue Cycloheptatrien-Derivate und ein Verfahren zu ihrer Herstellung.

Es sind bereits verschiedenartige Cycloheptatrien-Derivate bekannt. So kann beispielsweise durch Reaktion von Benzol mit Diazo-Essigsäureäthylester über die Zwischenstufe des Norcaradien-carbonsäure-äthylesters in einer Ringerweiterungsreaktion der Cycloheptatrien-carbonsäure-äthylester erhalten werden (Ber. dtsch. chem. Ges. 30, 632 (1897); Justus Liebigs Ann. Chem. 602, 94 (1957)). Weiterhin ist die Umsetzung von 1-Chlormethyl-3,5-cyclohexadien-1,2-dicarbonsäure-dimethylester mit Kalium-tert.-butylat ebenfalls unter Ringerweiterung zum Cycloheptatrien-1,6-dicarbonsäure-dimethylester bekannt (Helv. Chim. Acta 46, 2893 (1963)).

Durch photochemische Umwandlung aus Bicyclo-(3,2,0)-hepta-2,6-dien-1-carbonsäureäthylester (Tetrahedron Lett. 1973, 48) bzw. durch photochemische Reaktion von Diazo-essigsäuremethylester in Benzol (Justus Liebigs Ann. Chem. 1974, 835) können die Isomeren Cycloheptatrien-carbonsäureester erhalten werden.

Weiterhin ist die Herstellung von Cycloheptatrien-aldehyd durch Pyrolyse von Cyclooctatetraen-epoxid in einer Ringverengungsreaktion bei 260°C (J. Am. Chem. Soc. 84, 3104 (1962)) beschrieben worden.

Es wurden neue Cycloheptatrien-Derivate der Formel (I)

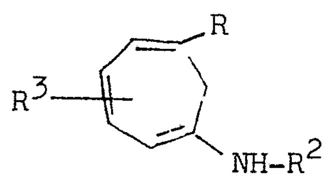

(I),

in der

R für Wasserstoff, $COR^1$ oder $NHR^2$ steht,

$R^1$ Alkyl, Cycloalkyl, Alkoxy oder Cycloalkoxy bedeutet,

$R^2$ für $CONH_2$, $COOR^4$, $CO\text{-}NHR^4$, $CO\text{-}NR^4R^5$, $COSR^4$ oder $COR^4$ steht und für den Fall, daß R $COR^1$ bedeutet, außerdem noch Wasserstoff sein kann,

$R^3$ Wasserstoff, Alkyl oder Cycloalkyl bedeutet und

$R^4$ und $R^5$ gleich oder verschieden sind und Alkyl, Cycloalkyl, Aryl oder Aralkyl bedeuten können,

gefunden.

Als Alkyl seien beispielsweise geradkettige oder verzweigte Kohlenwasserstoffreste mit 1 bis 4 Kohlenstoffatomen genannt, wie Methyl, Äthyl, Propyl, Isopropyl, Butyl oder Isobutyl. Bevorzugtes Alkyl ist Methyl oder Äthyl, ganz bevorzugt ist der Methylrest.

Als Cycloalkyl seie beispielsweise cyclische Kohlenwasserstoffreste mit 4 bis 6 Kohlenstoffatomen genannt, die gegebenenfalls durch eine oder mehrere Methylgruppen substituiert sein können, wie Cyclobutyl, Methylcyclobutyl, Cyclopentyl, Methyl-cyclopentyl, Cyclohexyl oder Methyl-cyclohexyl.

Als Alkoxy seien beispielsweise geradkettige oder verzweigte Reste mit 1 bis 4 Kohlenstoffatomen genannt, wie Methoxy, Äthoxy, Propoxy, Isopropoxy, Butoxy oder Isobutoxy.

Bevorzugte Alkoxy sind Methoxy und Äthoxy, ganz bevorzugt ist der Methoxyrest.

Als Cycloalkoxy seien beispielsweise Reste genannt, die sich von 4- bis 6-gliedrigen Ringen ableiten, die gegebenenfalls durch eine oder mehrere Methylgruppen substituiert sein können, wie Cyclobutoxy, Methylcyclobutoxy, Cyclopentyloxy, Methyl-cyclopentyloxy, Cyclohexyloxy oder Methyl-cyclohexyloxy.

Als Aryl seien aromatische Reste mit 6 bis 14 Kohlenstoffatomen genannt, beispielsweise Phenyl, Diphenyl, Naphthyl oder Anthryl. Bevorzugtes Aryl ist der Phenylrest.

Als Aralkyl seien Kohlenwasserstoffreste genannt, die im aliphatischen Teil 1 bis 2 Kohlenstoffatome und im aromatischen Teil 6 bis 14 Kohlenstoffatome enthalten, beispielsweise Benzyl, 2-Phenyl-äthyl, Naphthyl-methyl, 2-Naphthyl-äthyl, Anthryl-methyl oder 2-Anthryl-äthyl. Bevorzugtes Aralkyl ist der Benzylrest.

Selbstverständlich können die aromatischen Teile der Aryl- und Aralkyl-Reste gegebenenfalls durch niedere Alkylgruppen, Halogenatome, beispielsweise Fluor, Chlor oder Brom, Nitro, Cyano, Dialkylamino, Alkoxy, Carboxyalkyl, Carboxydialkylamido oder Acyl substituiert sein.

Als neue Cycloheptatrien-Derivate seien bevorzugt solche der Formel (I) genannt, bei denen

$R^3$ Wasserstoff bedeutet und

R, $R^1$ und $R^2$ die obengenannte Bedeutung besitzen.

Als neue Cycloheptatrien-Derivate seien besonders bevorzugt solche der Formel (I) genannt, bei denen

R für $COR^1$ steht,

$R^1$ für Methyl, Äthyl, Methoxy oder Äthoxy steht,

$R^2$ Wasserstoff, $CONH_2$, $COOR^{4'}$, $CONHR^{4'}$ oder $CONR^{4'}R^{5'}$ bedeutet, wobei $R^{4'}$ und $R^{5'}$ gleich oder verschieden sind und für Alkyl, Phenyl oder Benzyl stehen können, und

$R^3$ Wasserstoff bedeutet.

In ganz besonders bevorzugter Weise seien neue Cycloheptatrien-Derivate der Formel (I) genannt, bei denen

R für $COR^1$ steht,

$R^1$ für Methyl oder Methoxy,

$R^2$ für Wasserstoff oder $CONH_2$ und

$R^3$ für Wasserstoff steht.

Als Beispiele für die neuen Verbindungen der Formel (I) seien genannt:

1-Amino-6-acetyl-cyclohepta-1,3,5-trien, 6-Acetylcyclohepta-1,3,5-trienyl-harnstoff, 6-Carbomethoxy-cyclohepta-1,3,5-trienyl-harnstoff, 6-Propionyl-cyclohepta-1,3,5-trienyl-N'-phenyl-harnstoff, 6-Carbomethoxy-cyclohepta-1,3,5-trienyl-carbamidsäuremethylester, 6-Acetyl-cyclohepta-1,3,5-trienyl-acetamid, 6-Acetyl-2-methyl-cyclohepta-1,3,5-trienyl-harnstoff, 1-Amino-4-tert.-butyl-6-acetyl-cyclohepta-1,3,5-trien, 1-Amino-4-cyclohexyl-6-propionyl-cyclohepta-1,3,5-trien.

Die Erfindung betrifft weiterhin ein Verfahren zur Herstellung von Cycloheptatrien-Derivaten, das dadurch gekennzeichnet ist, daß man eine Cycloheptatriencarbonsäure der Formel (II)

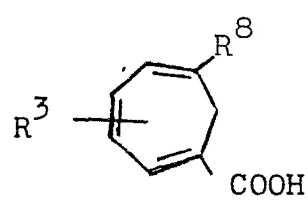

(II),

in der

$R^8$ für Wasserstoff, $COR^1$ oder COOH steht,

$R^1$ Alkyl, Cycloalkyl, Alkoxy oder Cycloalkoxy bedeutet, und

$R^3$ für Wasserstoff, Alkyl oder Cycloalkyl steht,

a) mit einem Halogenierungsmittel in Gegenwart eines Katalysators, gegebenenfalls in einem inerten organischen Lösungsmittel, im Temperaturbereich von 50 bis 100°C umsetzt,

b) das Umsetzungsprodukt nach a) in einem organischen Lösungsmittel löst und mit einem Azid umsetzt,

c) das Umsetzungsprodukt nach b) in einem inerten organischen Lösungsmittel unter Feuchtigkeitsausschluß auf 25 bis 140°C erwärmt und

d) das Umsetzungsprodukt aus c) mit einer Verbindung der Formel (III)

$$H - R^6 \qquad (III),$$

in der

$R^6$ für OH, $NH_2$, $OR^4$, $NHR^4$, $NR^4R^5$, $SR^4$ oder $O-COR^4$ steht, wobei $R^4$ und $R^5$ gleich oder verschieden sein können und Alkyl, Cycloalkyl, Aryl oder Aralkyl bedeuten können,

im Temperaturbereich von -10 bis +50°C, gegebenen-

0011670



falls in einem inerten organischen Lösungsmittel, umsetzt.

Cycloheptatrien-carbonsäuren der Formel (II), die als Ausgangsprodukte für das erfindungsgemäße Verfahren eingesetzt werden können, können durch Acetylierung von Cycloheptatrien-Derivaten der Formel (IV)

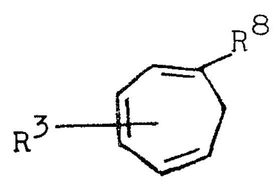

(IV),

in der

$R^8$ und $R^3$ die obengenannte Bedeutung besitzen,

mit einem Acetylierungsmittel in einem inerten Lösungsmittel im Temperaturbereich von -60 bis +150°C in Gegenwart eines Acylierungskatalysators und nachfolgender Umwandlung der Acetylgruppe in eine Carboxygruppe durch Oxidation mit Hypohalogenit gemäß der Patentanmeldung P 28 51 790.0 hergestellt werden.

Als Cycloheptatrien-carbonsäuren der Formel (II), die für das erfindungsgemäße Verfahren eingesetzt werden

können, seien beispielsweise genannt:

6-Acetyl-cyclohepta-1,3,5-trien-1-carbonsäure,
6-Propionyl-cyclohepta-1,3,5-trien-1-carbonsäure,
6-Cyclohexanoyl-cyclohepta-1,3,5-trien-1-carbonsäure,
Cyclohepta-1,3,5-trien-1,6-dicarbonsäure-monomethylester,
Cyclohepta-1,3,5-trien-1,6-dicarbonsäure-monobutylester,
Cyclohepta-1,3,5-trien-1,6-dicarbonsäure-monocyclohexylester,
6-Acetyl-2-methyl-cyclohepta-1,3,5-trien-1-carbonsäure,
6-Propionyl-3-cyclopentyl-cyclohepta-1,3,5-trien-1-carbonsäure,
6-Butylcarbonyl-2-methyl-cyclohepta-1,3,5-trien-1-carbonsäure,
Cyclohepta-1,3,5-trien-1,6-dicarbonsäure-monoäthylester.

Als Halogenierungsmittel für das erfindungsgemäße Verfahren seien beispielsweise die Säurehalogenide des Phosphors, des Schwefels oder des Kohlenstoffs, wie die Säurechloride und die Säurebromide, bevorzugt die Säurechloride, genannt. Beispielsweise sind für das erfindungsgemäße Verfahren geeignet: Phosphortrichlorid, Phosphortribromid, Phosphorpentachlorid, Phosphorpentabromid, Phosphoroxychlorid, Phosphoroxybromid, Thionylchlorid, Thionylbromid, Sulfurylchlorid, Sulfurylbromid oder Oxalylchlorid. Das bevorzugte Halogenierungsmittel ist das Thionylchlorid.

Als Katalysatoren für das erfindungsgemäße Verfahren seien beispielsweise Pyridin, Carbonsäureamide, wie

Dimethylformamid, Dimethylacetamid, N-Methyl-N-phenyl-acetamid, sowie tertiäre Amine, beispielsweise Triäthylamin, Dimethylbenzylamin oder Tributylamin, genannt.

Die Umsetzung von S offen der Formel (II) mit einem Halogenierungsmitte im erfindungsgemäßen Verfahren kann beispielsweise im Temperaturbereich von 50 bis 100°C durchgeführt werden.

Die Umsetzung mit einem Halogenierungsmittel im erfindungsgemäßen Verfahren kann mit oder ohne organische Lösungsmittel durchgeführt werden. Die Verfahrensvariante ohne organische Lösungsmittel ist beispielsweise dann vorteilhaft, wenn der Überschuß des Halogenierungsmittels als Lösungsmittel dient. Bei Verwendung eines organischen Lösungsmittels seien als Lösungsmittel solche genannt, die unter den Reaktionsbedingungen inert sind, wie beispielsweise: Tetrahydrofuran, Dioxan, Benzol, Toluol, Tetrachlorkohlenstoff, Chloroform, Methylenchlorid, Petroläther oder Äther.

Die Umsetzung mit einem Halogenierungsmittel wird gewöhnlich bei Normaldruck durchgeführt. Bei Verwendung eines niedrigsiedenden organischen Lösungsmittels kann jedoch auch ein höherer Druck, beispielsweise bis zu 10 bar, bevorzugt der Eigendruck des Lösungsmittels bei der jeweiligen Reaktionstemperatur angewendet werden.

Als Azid für die weitere Umsetzung sei beispielsweise ein Alkaliazid, wie Natriumazid oder Kaliumazid oder Trimethylsilylazid genannt.

Als organisches Lösungsmittel, in dem die weitere Umsetzung mit dem Azid stattfindet, sei beispielsweise Aceton, Acetonitril, Tetrahydrofuran oder Dioxan genannt.

Das Umsetzungsprodukt mit dem Azid wird danach bei höheren Temperaturen in einem inerten organischen Lösungsmittel unter Feuchtigkeitsausschluß erwärmt. Hierfür sei ein Temperaturbereich von 25 bis 140°C, bevorzugt 50 bis 130°C genannt. Als inertes organisches Lösungsmittel sei beispielsweise Benzol, Toluol, Xylol oder Cyclohexan genannt.

Zur Gewinnung der Stoffe der Formel (I) im erfindungsgemäßen Verfahren werden die Intermediärprodukte abschließend mit einer Verbindung der Formel (III) umgesetzt. Als Verbindungen der Formel (III) seien beispielsweise genannt:

Wasser, Ammoniak, niedere Alkohole, wie Methanol, Äthanol, Propanol, Isopropanol, Butanol, Isobutanol, Cyclopentylalkohol oder Cyclohexylalkohol, Phenol, substituierte Phenole, Naphthol, substituierte Naphthole, primäre und sekundäre Amine wie Methylamin, Dimethylamin, Äthylamin, Diäthylamin, Propylamin, Dipropylamin, Butylamin, Dibutylamin,

Cyclohexylamin, Anilin, N-Methyl-anilin, Benzylalkohol, N-Methyl-benzylamin, Mercaptane, wie Methylmercaptan, Äthylmercaptan, Butylmercaptan, Cyclopentylmercaptan, Cyclohexylmercaptan, Benzylmercaptan, Thiophenol, sowie Carbonsäuren, wie Essigsäure, Propionsäure, Buttersäure, Cyclopentylcarbonsäure, Cyclohexylcarbonsäure, Phenylessigsäure, Benzoesäure oder Naphthoesäure.

Die Umsetzung mit einer Verbindung der Formel (III) kann beispielsweise im Temperaturbereich von -10 bis +50°C, bevorzugt von 0 bis +30°C durchgeführt werden.

Die Umsetzung mit Verbindungen der Formel (III) im erfindungsgemäßen Verfahren wird in einem inerten organischen Lösungsmittel durchgeführt. Als solche seien Kohlenwasserstoffe, wie Petroläther, Cyclohexan, Benzol, Toluol, Xylol, Äther, wie Diäthyläther, Dioxan oder Tetrahydrofuran, oder chlorierte Kohlenwasserstoffe wie Methylenchlorid, Chloroform oder Chlorbenzol, sowie Dimethylsulfoxid, sowie Essigsäureäthylester oder Dimethylformamid genannt. Gegebenenfalls kann die Umsetzung mit einer Verbindung der Formel (III) durch Zusatz einer kleinen Menge Mineralsäure beschleunigt werden.

Die im erfindungsgemäßen Verfahren als Intermediärstufen auftretenden Cycloheptatrien-Derivate der Formel (V)

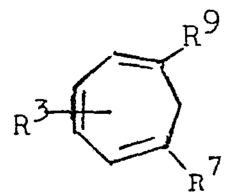

(V),

in der

$R^9$ für Wasserstoff, $COR^1$ oder $R^7$ steht,

$R^1$ und $R^3$ die obengenannte Bedeutung haben und

$R^7$ für -COCl, $-CON_3$ oder -NCO steht,

sind ebenfalls neu.

Die Erfindung betrifft daher weiterhin die neuen Verbindungen der Formel (V).

Als Beispiele für Verbindungen der Formel (V) seien genannt:

6-Acetyl-cyclohepta-1,3,5-trien-carbonsäureazid, 6-Propionyl-cyclohepta-1,3,5-trien-carbonsäureazid, 6-Carbomethoxy-cyclohepta-1,3,5-trien-carbonsäureazid, 2-Methyl-6-acetyl-cyclohepta-1,3,5-trien-carbonsäureazid, 4-tert.-Butyl-6-carbocyclohexyloxy-cyclohepta-1,3,5-trien-carbonsäureazid, 6-Acetyl-cyclohepta-1,3,5-trienyl-isocyanat, 6-Cyclohexanoyl-cyclohepta-1,3,5-trienyl-isocyanat, 6-Carboäthoxy-cyclohepta-1,3,5-trienyl-isocyanat, 4-Cyclopentyl-6-carbobutyloxy-cyclohepta-1,3,5-trienyl-isocyanat, 6-Acetyl-cyclohepta-1,3,5-trien-carbonsäurechlorid, 6-Carbopropoxy-cyclohepta-1,3,5-trien-carbonsäurechlorid, 4-Äthyl-6-acetyl-cyclohepta-1,3,5-triencarbonsäurechlorid.

Es wurde weiterhin gefunden, daß das Cyclohepta-1,3,5-trien-1,6-dicarbonsäuredichlorid mit Lithium-Aluminium-tris-(tert.-butoxy)-hydrid in einem Äther als Lösungsmittel bei einer Temperatur von -90°C bis 0°C, bevorzugt -80°C bis -20°C, zum Cyclohepta-1,3,5-trien-1,6-dialdehyd umgesetzt werden kann.

Das erfindungsgemäße Verfahren ist dadurch besonders fortschrittlich, daß es eine große Zahl neuer Verbindungen mit interessanten Eigenschaften zugänglich macht.

Während nach dem Stand der Technik Cycloheptatrien-Derivate durch photochemische Umsetzungen und durch Umwandlung von polycyclischen Verbindungen nur in einem kleinen Maßstab zugänglich waren, ist es nach dem erfindungsgemäßen Verfahren völlig überraschend möglich, die offenbarten Reaktionen auch im größeren Maßstab durchzuführen. Es ist weiterhin völlig überraschend, daß das Cycloheptatrien-Gerüst mit seinen nicht durch aromatische Resonanz stabilisierten Doppelbindungen bei den verschiedenen Stufen des erfindungsgemäßen Verfahrens intakt bleibt.

Die Stoffe, die durch das erfindungsgemäße Verfahren erhalten werden können, sind wertvolle Zwischenprodukte für die Herstellung von Geruchsstoffen und Aromen,

Pharmaka und Pflanzenschutzmitteln, wie Insektiziden, Herbiziden oder Fungiziden. Teilweise haben sie selbst pharmakologische, insektizide, herbizide bzw. fungizide Wirksamkeit. Die neuen Stoffe der vorliegenden Patentanmeldung sind ferner, soweit sie difunktionell sind, als Monomere oder Comonomere für Polykondensate mit wertvollen Eigenschaften einsetzbar. Die neuen Verbindungen der vorliegenden Patentanmeldung sind weiterhin aufgrund der Doppelbindungen des Cycloheptatrien-Systems als Monomere oder Comonomere für Polymerisate mit wertvollen Eigenschaften einsetzbar.

Die Erfindung wird durch die nachfolgenden Ausführungsbeispiele erläutert, ohne daß sie durch diese eingegrenzt würde.

Beispiel 1

6-Carbomethoxy-cyclohepta-1,3,5-trienyl-isocyanat

Eine Lösung von 9 g (0,05 Mol) Cyclohepta-1,3,5-trien-1,6-dicarbonsäure-monomethylester in 100 ml absolutem Tetrahydrofuran wird mit 10 ml $SOCl_2$ und einer katalytischen Menge Dimethylformamid versetzt und 30 Min. unter Feuchtigkeitsausschluß unter Rückfluß erhitzt. Der nach Einengen der Lösung resultierende ölige Rückstand wird in 300 ml absolutem Aceton gelöst. Zu der auf 0°C gekühlten Lösung läßt man eine Lösung von 6,5 g (0,1 Mol) Natriumazid in 5 ml Wasser zutropfen. Nach 30-minütigem Rühren bei Raumtemperatur und Zugabe von Eiswasser wird mit Äther dreimal extrahiert. Trocknen mit $MgSO_4$ und Einengen der Ätherphase liefert ein braunes Öl, das an $Al_2O_3$ basisch, Aktivitätsstufe V (Säule: 50/4 cm) mit Toluol chromatographiert wird. Die gelbe Fraktion wird eingeengt und ergibt das 6-Carbomethoxy-cyclohepta-1,3,5-trien-carbonsäureazid als gelbe Festsubstanz. Ausbeute 8 g = 87 % der theoretischen Ausbeute; Fp. 74°C (unter Zersetzung).
Die von der Chromatographie resultierende Toluollösung (ca. 1 l) des Azids wird 2 h am Rückfluß unter Feuchtigkeitsausschluß erhitzt, und ergibt das 6-Carbomethoxy-cyclohepta-1,3,5-trienyl-isocyanat. Ausbeute 6,6 g = 95 % der theoretischen Ausbeuten, bezogen auf das eingesetzte Azid.

Beispiel 2

6-Carbomethoxy-cyclohepta-1,3,5-trienyl-harnstoff

Beim Einleiten von Ammoniak in die Toluollösung des Isocyanats nach Beispiel 1 fällt das Harnstoffderivat als gelbe Festsubstanz aus. Gelbe Nadeln (aus Methanol) vom Fp. 148 - 149°C (Zersetzung); Ausbeute 6,7 g = 70 %, bezogen auf den Cyclohepta-1,3,5-trien-1,6-dicarbonsäure-monomethylester (s. Beispiel 1).

Beispiel 3

6-Carbomethoxy-cyclohepta-1,3,5-trienyl-amin

Zu 1,9 g (10 mMol) Isocyanat nach Beispiel 1 werden 30 ml destilliertes Wasser gegeben und soviel Dimethylsulfoxid, bis die Lösung homogen ist. Unter Zusatz einer katalytischen Menge HCl wird die Lösung 3 h bei Raumtemperatur gerührt. Man verdünnt mit 200 ml Wasser und extrahiert mit Äther. Die Ätherphase wird mit Wasser gewaschen und getrocknet ($Na_2SO_4$). Chromatographie des Ätherrückstandes an $SiO_2$ mit Äther-Pentan (1:1) liefert in der 2. Fraktion das 6-Carbomethoxy-cyclohepta-1,3,5-trienyl-amin als orange-rotes Öl; Ausbeute: 0,82 g = 50 % der theoretischen Ausbeute.

Beispiel 4

Cyclohepta-1,3,5-trien-1,6-dicarbonsäuredichlorid

100 g (0,56 Mol) Cyclohepta-1,3,5-trien-1-dicarbonsäure versetzt man mit 600 g $SOCl_2$ und einer katalytischen Menge Pyridin (1 - 2 ml) und erhitzt unter Rückfluß, bis sich die gesamte Carbonsäure aufgelöst hat. Überschüssiges $SOCl_2$ wird abdestilliert und der Rückstand im Ölpumpenvakuum destilliert.

Die Fraktion von 130 bis 137°C bei 0,7 Torr besteht aus reinem Disäurechlorid. Fp: 63°C; Ausbeute: 97,5 g (81 % der theoretischen Ausbeute).

| | | | |
|---|---|---|---|
| C, H-Analyse: | Ber.: | C 49,80 %, | H 2,79 % |
| | Gef.: | C 49,56 %, | H 3,05 % |

Beispiel 5

Cyclohepta-1,3,5-trien-1,6-dicarbonsäuredimethylester

Zu der Lösung von 21,7 g (0,1 Mol) Cyclohepta-1,3,5-trien-1,6-dicarbonsäuredichlorid in 50 ml absolutem Tetrahydrofuran werden unter Eiskühlung 50 ml absolutes Methanol tropfenweise zugefügt (HCl-Entwicklung!). Man läßt eine Stunde bei Raumtemperatur rühren und engt die braune Lösung ein. Unter Zusatz von Aktivkohle wird aus Methanol umkristallisiert, man erhält 14,5 g Diester vom Fp. 53 - 54°C in 72 % der theoretischen Ausbeute.

Beispiel 6

Cyclohepta-1,3,5-trien-1,6-dialdehyd

141 g (0,65 Mol) Cycloheptatrien-1,6-dicarbonsäuredichlorid werden in 700 ml absolutem Tetrahydrofuran (THF) gelöst und auf -78°C gekühlt. Unter Inertgas-Atmosphäre läßt man innerhalb von 1 1/2 Stunden 330 g (1,3 Mol) $LiAlH[OC(CH_3)_3]_3$, gelöst in 1 l absolutem THF, zutropfen. Man läßt eine Stunde bei -78°C nachrühren und anschließend auf 0°C auftauen. Das Reaktionsgemisch wird mit 1,5 l gesättigter Kalium-Natriumtartrat-Lösung extrahiert. Die wäßrige Phase wäscht man noch einmal mit Äther. Die vereinigten organischen Phasen trocknet man über $MgSO_4$ und engt ein. Der Rückstand wird in absolutem Äthanol gelöst und die Lösung mit Aktivkohle gereinigt. Nach Abzug des Lösungsmittels kristallisiert man aus Essigester/Pentan um. Ausbeute 58,7 g = 61 % der theoretischen Ausbeute an Cyclohepta-1,3,5-trien-1,6-dialdehyd.

Patentansprüche

1. Cycloheptatrien-Derivate der Formel

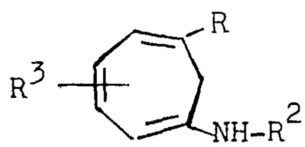

in der

R für Wasserstoff, $COR^1$ oder $NHR^2$ steht,

$R^1$ Alkyl, Cycloalkyl, Alkoxy oder Cycloalkoxy bedeutet,

$R^2$ für $CONH_2$, $COOR^4$, $CO\text{-}NHR^4$, $CO\text{-}NR^4R^5$, $COSR^4$ oder $COR^4$ steht und für den Fall, daß R $COR^1$ bedeutet, außerdem noch Wasserstoff sein kann,

$R^3$ Wasserstoff, Alkyl oder Cycloalkyl bedeutet und

$R^4$ und $R^5$ gleich oder verschieden sind und Alkyl, Cycloalkyl, Aryl oder Aralkyl bedeuten können.

2. Cycloheptatrien-Derivate nach Anspruch 1, dadurch gekennzeichnet, daß $R^3$ Wasserstoff ist und R, $R^1$ und $R^2$ die in Anspruch 1 angegebene Bedeutung haben.

3. Cycloheptatrien-Derivate nach Anspruch 1, dadurch gekennzeichnet, daß R für $COR^1$ steht, $R^1$ für Methyl, Äthyl, Methoxy und Äthoxy steht, $R^2$ Wasserstoff, $CONH_2$, $COOR^{4'}$, $CONHR^{4'}$ oder $CONR^{4'}R^{5'}$ bedeutet, wobei $R^{4'}$ und $R^{5'}$ gleich oder verschieden sind und für Alkyl, Phenyl oder Benzyl stehen können, und $R^3$ Wasserstoff ist.

4. Cycloheptatrien-Derivate nach Anspruch 1, dadurch gekennzeichnet, daß R für $COR^1$ steht, $R^1$ für Methyl oder Methoxy, $R^2$ für Wasserstoff oder $CONH_2$ und $R^3$ für Wasserstoff steht.

5. Cycloheptatrien-Derivate der Formel

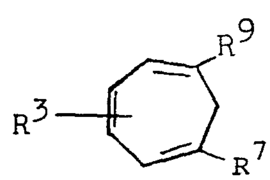
,

in der

$R^9$ für Wasserstoff, $COR^1$ oder $R^7$ steht,

$R^1$ und $R^3$ die in Anspruch 1 genannte Bedeutung haben und $R^7$ für -COCl, $-CON_3$ oder -NCO steht.

6. Verfahren zur Herstellung von Cycloheptatrien-Derivaten, dadurch gekennzeichnet, daß man eine Cycloheptatrien-carbonsäure der Formel

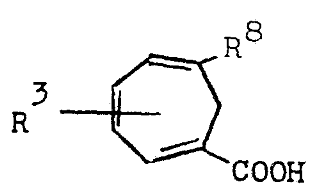

in der

$R^8$ für Wasserstoff, $COR^1$ oder COOH steht,

$R^1$ Alkyl, Cycloalkyl, Alkoxy oder Cycloalkoxy bedeutet und

$R^3$ für Wasserstoff, Alkyl oder Cycloalkyl steht,

a) mit einem Halogenierungsmittel in Gegenwart eines Katalysators, gegebenenfalls in einem inerten organischen Lösungsmittel im Temperaturbereich von 50 bis 100°C umsetzt,

b) das Umsetzungsprodukt nach a) in einem organischen Lösungsmittel löst und mit einem Azid umsetzt,

c) das Umsetzungsprodukt nach b) in einem inerten organischen Lösungsmittel unter Feuchtigkeitsausschluß auf 25 bis 140°C erwärmt und

d) das Umsetzungsprodukt nach c) mit einer Verbindung der Formel

$$H - R^6,$$

in der

$R^6$ für OH, $NH_2$, $OR^4$, $NHR^4$, $NR^4R^5$, $SR^4$ oder $O\text{-}COR^4$ steht, wobei $R^4$ und $R^5$ gleich oder verschieden sein können und Alkyl, Cycloalkyl, Aryl oder Aralkyl bedeuten können,

im Temperaturbereich von -10 bis +50°C, gegebenenfalls in einem inerten organischen Lösungsmittel, umsetzt.

7. Verfahren nach Anspruch 8, dadurch gekennzeichnet, daß man als Halogenierungsmittel Thionylchlorid einsetzt.

8. Verfahren zur Herstellung des Cyclohepta-1,3,5-trien-1,6-dialdehyds, dadurch gekennzeichent, daß man Cyclohepta-1,3,5-trien-1,6-dicarbonsäuredichlorid mit Lithium-Aluminium-tris-(tert,-butoxy)-hydrid in einem Äther als Lösungsmittel bei einer Temperatur von -90°C bis 0°C umsetzt.